(19) Europäisches Patentamt
European Patent Office
Office européen des brevets

(11) EP 4 191 729 B1

(12) EUROPEAN PATENT SPECIFICATION

(45) Date of publication and mention of the grant of the patent:
**06.11.2024 Bulletin 2024/45**

(21) Application number: **22776117.8**

(22) Date of filing: **24.03.2022**

(51) International Patent Classification (IPC):
***H01M 10/0567** (2010.01)* ***H01M 10/052** (2010.01)*
***H01M 4/525** (2010.01)* ***H01M 4/505** (2010.01)*
***H01M 4/58** (2010.01)* ***H01M 10/0525** (2010.01)*
***H01M 10/0568** (2010.01)* ***C07D 233/88** (2006.01)*

(52) Cooperative Patent Classification (CPC):
**C07D 233/88; H01M 4/505; H01M 4/525; H01M 4/5825; H01M 10/0525; H01M 10/0567; H01M 10/0568;** H01M 2300/0037; Y02E 60/10

(86) International application number:
**PCT/KR2022/004127**

(87) International publication number:
**WO 2022/203417 (29.09.2022 Gazette 2022/39)**

(54) **NON-AQUEOUS ELECTROLYTE COMRISING AN AMINOIMIDAZOLE ADDITIVE FOR LITHIUM SECONDARY BATTERY, AND LITHIUM SECONDARY BATTERY COMPRISING SAME**

WASSERFREIER AMINOIMIDAZOL-ADDITIV ENTHALTENDER ELEKTROLYT FÜR LITHIUMSEKUNDÄRBATTERIE UND MIT IHM VERSEHENE LITHIUMSEKUNDÄRBATTERIE

ÉLECTROLYTE NON AQUEUX COMRENANT UN ADDITIF AMINOIMIDAZOLE POUR BATTERIE SECONDAIRE AU LITHIUM, ET BATTERIE SECONDAIRE AU LITHIUM LE COMPRENANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.03.2021 KR 20210037733**

(43) Date of publication of application:
**07.06.2023 Bulletin 2023/23**

(73) Proprietor: **LG Energy Solution, Ltd.**
**Seoul 07335 (KR)**

(72) Inventors:
- **OH, Jeong Woo**
**Daejeon 34122 (KR)**
- **PARK, Sung Guk**
**Daejeon 34122 (KR)**
- **LEE, Chul Haeng**
**Daejeon 34122 (KR)**

(74) Representative: **Hoffmann Eitle**
**Patent- und Rechtsanwälte PartmbB**
**Arabellastraße 30**
**81925 München (DE)**

(56) References cited:
**JP-A- 2003 123 834**
**JP-A- 2004 207 451**
**JP-A- 2007 095 983**
**KR-A- 20170 048 915**
**KR-B1- 101 459 402**

- **No further relevant documents disclosed**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

## Description

### TECHNICAL FIELD

This application claims priority from Korean Patent Application No. 10-2021-0037733 filed on March 24, 2021.

The present invention relates a non-aqueous electrolyte solution for a lithium secondary battery, and a lithium secondary battery including the same.

### BACKGROUND ART

A lithium secondary battery is generally manufactured by interposing a separator between a positive electrode including a positive electrode active material composed of a transition metal oxide containing lithium, and a negative electrode including a negative electrode active material capable of storing lithium ions, thereby providing an electrode assembly, inserting the electrode assembly into a battery case, injecting thereto a non-aqueous electrolyte solution, which is a medium for transferring the lithium ions, and then sealing the battery case.

A lithium secondary battery may be miniaturized, and has high energy density and working voltage, thereby being applied in various fields including mobile devices, electronic products, electric vehicles, and the like. As the field of application of a lithium secondary battery becomes diverse, required physical properties conditions of the lithium secondary battery are also increasing, and particularly, there is a demand for the development of a lithium secondary battery which may be stably driven even under high-temperature conditions.

Meanwhile, when a lithium secondary battery is driven under high-temperature conditions, $PF_6^-$ anions may be thermally decomposed from a lithium salt, such as $LiPF_6$, which is included in an electrolyte solution, so that a Lewis acid such as $PF_5$ may be generated, and the Lewis acid may react with moisture to generate HF. A decomposition product such as $PF_5$ and HF may destroy a film formed on the surface of an electrode, and may cause a decomposition reaction of an organic solvent. In addition, the decomposition product may react with decomposition products of a positive electrode active material to elute transition metal ions, and the eluted transition metal ions may be electro-deposited on a negative electrode to destroy a film formed on the surface of the negative electrode.

When an electrolyte decomposition reaction continues on the destroyed film as described above, the performance of a battery is further degraded, so that there is a demand for the development of a secondary battery which may maintain excellent performance even under high-temperature conditions. JP 2003 123834 A discloses an electrolyte with a watersoluble N-containing heterocyclic compound such as an imidazole, a triazole or a pyrazole added into an aqueous solution of an organic or inorganic acid. KR 101 459 402 B1 describes an electrolyte solution for a secondary battery comprising a nonaqueous solvent and an ionic salt, as well as a first additive such as a vinylene carbonate compound and a second additive such as a siloxane-based compound containing at least one unsaturated group. KR 2017 0048915 A discloses a lithium secondary battery in which a nonaqueous electrolyte which comprises a phosphorane-based compound having a sulfonic acid salt as an additive is used. JP 2004 207451 A and JP 2007 095983 A further describe an electrolytic solution for an electrochemical device with a quaternized and substituted imidazole.

### DISCLOSURE OF THE INVENTION

### TECHNICAL PROBLEM

An aspect of the present invention provides a non-aqueous electrolyte solution and a lithium secondary battery including the same, wherein a decomposition product generated by a lithium salt inside the electrolyte solution of the lithium secondary battery is effectively removed, and also, a decomposition reaction of positive/negative electrodes and an organic solvent is suppressed.

### TECHNICAL SOLUTION

According to an aspect of the present invention, there is provided a non-aqueous electrolyte solution for a lithium secondary battery, the electrolyte solution including a lithium salt, an organic solvent, and a first additive which is a compound represented by Formula 1 below.

[Formula 1]

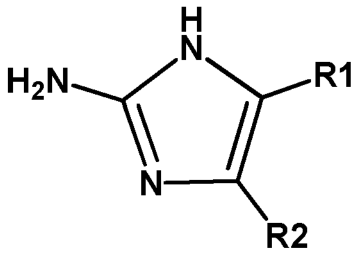

In Formula 1, R1, and R2 are each independently hydrogen, or an alkyl group having 1 to 5 carbon atoms.

According to another aspect of the present invention, there is provided a lithium secondary battery including a positive electrode including a positive electrode active material, a negative electrode including a negative electrode active material, a separator interposed between the positive electrode and the negative electrode, and the non-aqueous electrolyte solution.

## ADVANTAGEOUS EFFECTS

In order to solve the above problems, the present invention may provide a non-aqueous electrolyte solution for a lithium secondary battery including an electrolyte solution additive for a secondary battery which forms a robust film on surfaces of positive/negative electrodes, and also, has an excellent effect of removing a decomposition product generated from a lithium salt.

In addition, the present invention may provide a lithium secondary battery with improved high-temperature storage properties and lifespan properties of a battery by including the non-aqueous electrolyte solution for a lithium secondary battery.

## BEST MODE FOR CARRYING OUT THE INVENTION

Hereinafter, the present invention will be described in more detail.

In general, anions of a lithium salt, such as $LiPF_6$, which is widely used in a lithium secondary battery, may form a decomposition product such as hydrogen fluoride (HF) and $PF_5$ by thermal decomposition or moisture. Such a decomposition product has properties of an acid, and deteriorates a film or the surface of an electrode in a battery.

Specifically, the decomposition product easily elutes a transition metal constituting a positive electrode into an electrolyte solution, and the eluted transition metal ions move to a negative electrode through the electrolyte solution, and then electro-deposited on a solid electrolyte interphase (SEI) film formed on the negative electrode to cause an additional electrolyte decomposition reaction.

Such a series of reactions reduce the amount of available lithium ions in the battery, which causes the deterioration in battery capacity, and also causes an additional electrolyte solution decomposition reaction, resulting an increase in resistance.

In addition, when forming a positive electrode, if metal impurities are included in the electrode, the metal impurities are eluted from the positive electrode during an initial charging and moved to a negative electrode, and then electro-deposited on the surface of the negative electrode as metal ions. The electro-deposited metal ions grow into dendrites and cause an internal short circuit of the battery, and thus, become a major cause of low voltage failure.

In the present invention, eluted metal ions causing the deterioration and failure behavior may be removed from the inside of a battery to prevent the metal ions from being electro-deposited on the surface of an electrode, a robust film may be formed on surfaces of positive/negative electrodes to suppress the elution of a transition metal and control an electro-deposition reaction in the negative electrode, and an electrochemical decomposition reaction of an electrolyte solution may be controlled to control a gaseous by-product generated by the decomposition of the electrolyte solution so as to improve the durability of the battery.

The present inventors have used a compound represented by Formula 1 below as an additive to a non-aqueous electrolyte solution, and though which they have confirmed that it is possible to effectively remove a decomposition product generated from a lithium salt, and also to form a film on positive/negative electrodes to prevent a continuous decomposition reaction of the positive electrode and an organic solvent.

Specifically, it has been found that the compound represented by Formula 1 includes a primary amine and a secondary amine, and thus, may more effectively neutralize the Lewis acidity of the electrolyte solution, through which an electrolyte decomposition reaction and transition metal elution may be controlled, and since imidazole substituted with an amino group induces a Lewis acid-base reaction, there is an effect of controlling a negative electrode etching

reaction by HF. In addition, it has been confirmed that since a nitrogen atom-based solid electrolyte interface (SEI) and a cathode electrolyte interface (CEI) are formed through the amino group, the thermal stability of the film is improved, and ultimately, the high-temperature durability of the battery may be improved.

## Non-aqueous electrolyte solution

### (1) Additive

The non-aqueous electrolyte solution of the present invention includes a first additive which is a compound represented by Formula 1 below.

[Formula 1]

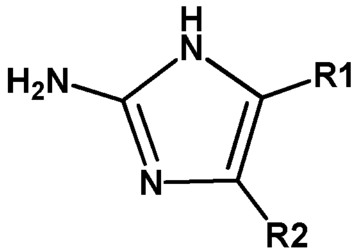

In Formula 1, R1, and R2 are each independently hydrogen, or an alkyl group having 1 to 5 carbon atoms.
In an embodiment of the present invention, when R1 and R2 are amino groups, the electron density of nitrogen atoms is decreased to rather lower the high-temperature stability, so that it is preferable that R1 and R2 are each hydrogen or an alkyl group having 1 to 5 carbon atoms, and more preferably, R1 and R2 may each be hydrogen.
In an embodiment of the present invention, the compound represented by Formula 1 may be represented by Formula 1-1 below.

[Formula 1-1]

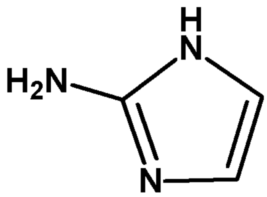

In an embodiment of the present invention, the content of the first additive is 0.1 wt% to 5 wt% based on the total weight of the non-aqueous electrolyte solution, preferably 0.1 wt% to 3 wt%, and more preferably 0.5 wt% to 1 wt%.
When the content of the first additive is in the above range, there is an effect in that the first additive participates in the formation of electrochemical positive/negative electrode films while appropriately controlling the Lewis acidity of the electrolyte solution.
Specifically, when the content of the first additive is less than 0.1 wt%, the first additive cannot participate in an electrochemical decomposition reaction, so that the effect of forming a film protecting the interface between positive/negative electrodes and an electrolyte solution is reduced, and when greater than 5 wt%, the first additive excessively participates in a decomposition reaction at the interface, so that film resistance is excessively increased to reduce the permeability of lithium ions under high-rate and low-temperature conditions, which may cause a problem of increasing the resistance of a battery. Particularly, in terms of decreasing the initial resistance of a battery, it is preferable that the content of the first additive is 3 wt% or less.
In an embodiment of the present invention, the non-aqueous electrolyte solution further includes one or more second additives selected from vinylene carbonate and vinylethylene carbonate, and preferably, includes vinylene carbonate as the second additive.
When the compound represented by Formula 1 is used together with vinylene carbonate and/or vinylethylene carbonate, polyethylene oxide (PEO) and a nitrogen atom-based SEI are formed to enhance the durability of a film.
In an embodiment of the present invention, the content of the second additive is 0.1 wt% to 5 wt% based on the total weight of the non-aqueous electrolyte solution, preferably 0.2 wt% to 3 wt%, and more preferably 0.3 wt% to 1 wt%.
When the content of the second additive is 0.1 wt% or greater, the second additive may participate in a SEI

film formation reaction to achieve an effect of enhancing durability. However, when greater than 5 wt%, a film including PEO having relatively low ion transfer properties is formed thick, so that it is not preferable in that resistance increases.

In an embodiment of the present invention, the weight ratio of the first additive and the second additive may be 1:0.2 to 1:1.5, preferably 1:0.5 to 1:1. When the first additive and the second additive are included in the above weight ratio, there is an effect of achieving suitable SEI durability and ion transport properties.

The non-aqueous electrolyte solution of the present invention may optionally further include a third additive, if necessary, in order to prevent a non-aqueous electrolyte from decomposing in a high voltage environment, thereby causing electrode collapse, or to further improve low-temperature high-rate discharge properties, high-temperature stability, overcharge prevention, the effect of suppressing battery expansion at high temperatures, and the like.

The third additive may be one or more selected from a halogen-substituted carbonate-based compound, a sultone-based compound, a sulfate-based compound, a phosphorus-based compound, a nitrile-based compound, an amine-based compound, a silane-based compound, a benzene-based compound, and a lithium salt-based compound.

The halogen-substituted carbonate-based compound may be fluoroethylene carbonate (FEC).

The sultone-based compound is a material capable of forming a stable SEI film by a reduction reaction on the surface of a negative electrode, and may be one or more compounds selected from 1,3-propane sultone (PS), 1,4-butane sultone, ethene sulfone, 1,3-propene sultone (PRS), 1,4-butene sultone, and 1-methyl-1,3-propene sultone, and specifically, may be 1,3-propane sultone (PS).

The sulfate-based compound is a material which may be electrically decomposed on the surface of a negative electrode, thereby forming a stable SEI thin film which is not cracked even during high-temperature storage, and may be one or more selected from ethylene sulfate (ESA), trimethylene sulfate (TMS), or methyl trimethylene sulfate (MTMS) .

The phosphorus-based compound may be one or more selected from lithium difluoro(bisoxalato)phosphate, lithium difluorophosphate, tris(trimethyl silyl)phosphate, tris(trimethyl silyl)phosphite, tris(2,2,2-trifluoroethyl)phosphate, and tris(trifluoroethyl)phosphite.

The nitrile-based compound may be one or more selected from succinonitrile, adiponitrile, acetonitrile, propionitrile, butyronitrile, valeronitrile, caprylonitrile, heptanenitrile, cyclopentane carbonitrile, cyclohexane carbonitrile, 2-fluorobenzonitrile, 4-fluorobenzonitrile, difluorobenzonitrile, trifluorobenzonitrile, phenylacetonitrile, 2-fluorophenylacetonitrile, and 4-fluorophenylacetonitrile.

The amine-based compound may be one or more selected from triethanolamine and ethylenediamine, and the silane-based compound may be tetravinylsilane.

The benzene-based compound may be one or more selected from monofluorobenzene, difluorobenzene, trifluorobenzene, and tetrafluorobenzene.

The lithium salt-based compound is a compound different from a lithium salt included in the electrolyte, and may be one or more compounds selected from $LiPO_2F_2$, lithium bisoxalatoborate ($LiB(C_2O_4)_2$) (LiBOB), lithium tetraphenylborate, and lithium tetrafluoroborate ($LiBF_4$).

Meanwhile, the content of the third additive may be 0.1 wt% to 10 wt%, preferably 1 wt% to 5 wt%, based on the total weight of the non-aqueous electrolyte solution. When the content of the third additive is less than 0.1 wt%, the effect of improving the low-temperature capacity of a battery as well as the high-temperature storage properties and high-temperature lifespan properties of the same is insignificant, and when greater than 10 wt%, there is a possibility in that side reactions in an electrolyte solution may excessively occur during charging and discharging of the battery. Particularly, when additives for forming the SEI film are added in excess, the additives may not be sufficiently decomposed at a high temperature, and thus, may be present as unreacted substances or precipitated in an electrolyte solution at room temperature. Accordingly, a side reaction causing the lifespan or resistance properties of the battery to degrade may occur.

(2) Organic solvent

The non-aqueous electrolyte solution of the present invention includes an organic solvent.

As the organic solvent, various organic solvents typically used in a lithium electrolyte may be used without limitation. For example, the organic solvent may be a cyclic carbonate-based solvent, a linear carbonate-based solvent, a linear ester-based solvent, a cyclic ester-based solvent, a nitrile-based solvent, or a mixture thereof, and preferably, may include a cyclic carbonate-based solvent and a linear carbonate-based solvent. In this case, the volume ratio of the cyclic carbonate-based solvent and the linear carbonate-based solvent may be 3:7 to 2:8.

The cyclic carbonate-based solvent is a high-viscosity organic solvent having a high dielectric constant, and thus, may dissociate a lithium salt in an electrolyte well, and may be one or more selected from the group consisting of ethylene carbonate (EC), propylene carbonate (PC), 1,2-butylene carbonate, 2,3-butylene carbonate, 1,2-pentylene carbonate, 2,3-pentylene carbonate, and vinylene carbonate, and specifically, may include ethylene carbonate (EC) .

In addition, the linear carbonate-based solvent is a low-viscosity, low-dielectric constant organic solvent, and may be one or more selected from the group consisting of dimethyl carbonate (DMC), diethyl carbonate (DEC), dipropyl

carbonate, ethylmethyl carbonate (EMC), methylpropyl carbonate, and ethylpropyl carbonate, and specifically, may include ethylmethyl carbonate (EMC).

In order to prepare an electrolyte solution having high ion conductivity, it is preferable that a mixture of a cyclic carbonate-based solvent and a linear carbonate-based solvent is used as the organic solvent.

The linear ester-based solvent may be one or more selected from methyl acetate, ethyl acetate, propyl acetate, methyl propionate, ethyl propionate, propyl propionate, and butyl propionate.

The cyclic ester-based solvent may be one or more selected from γ-butyrolactone, γ-valerolactone, γ-caprolactone, σ-valerolactone, and ε-caprolactone.

The nitrile-based solvent may be one or more selected from succinonitrile, acetonitrile, propionitrile, butyronitrile, valeronitrile, caprylonitrile, heptanenitrile, cyclopentane carbonitrile, cyclohexane carbonitrile, 2-fluorobenzonitrile, 4-fluorobenzonitrile, difluorobenzonitrile, trifluorobenzonitrile, phenylacetonitrile, 2-fluorophenylacetonitrile, and 4-fluorophenylacetonitrile, and preferably, may be succinonitrile.

The remainder of the total weight of the non-aqueous electrolyte solution except for the contents of other components, for example, the additive and the lithium salt, other than the organic solvent, may be the organic solvent unless otherwise stated.

### (3) Lithium salt

The non-aqueous electrolyte solution of the present invention includes a lithium salt.

As the lithium salt, any lithium salt typically used in an electrolyte solution for a lithium secondary battery may be used without imitation, and specifically, the lithium salt may include $Li^+$ as positive ions, and one or more selected from $(CF_2SO_2)_2N^-$, $(CF_3SO_2)_2N^-$, $(FSO_2)_2N^-$, $BF_2C_2O_4^-$, $BC_4O_8^-$, $BF_2C_2O_4CHF^-$, $PF_4C_2O_4^-$, $PF_2C_4O_8^-$, $PO_2F_2^-$, $(CF_3)_2PF_4^-$, $(CF_3)_3PF_3^-$, $(CF_3)_4PF_2^-$, $(CF_3)_5PF^-$, $(CF_3)_6P^-$, $C_4F_9SO_3^-$, $CF_3CF_2SO_3^-$, $CF_3CF_2(CF_3)_2CO^-$, $(CF_3SO_2)_2CH^-$, $CF_3(CF_2)_7SO_3^-$, and $SCN^-$ as negative ions.

Specifically, the lithium salt may be one or more selected from $LiPF_6$, $LiClO_4$, $LiBF_4$, $LiN(FSO_2)_2$(LiFSI), LiTFSI, lithium bis(pentafluoroethanesulfonyl)imide (LiBETI), $LiSO_3CF_3$, $LiPO_2F_2$, lithium bis (oxalate) borate (LiBOB), lithium difluoro(oxalate)borate (LiFOB), lithium difluoro(bisoxalato) phosphate (LiDFBP), lithium tetrafluoro(oxalate) phosphate (LiTFOP), and lithium fluoromalonato(difluoro) borate (LiFMDFB), and preferably, may be $LiPF_6$.

In an embodiment of the present invention, the concentration of a lithium salt in the electrolyte may be 0.3 to 3.0 M, specifically 0.5 M to 2.0 M, more specifically 0.5 M to 1.5 M. When the concentration of a lithium salt is in the above range, an effect of improving low-temperature output and improving cycle properties is sufficiently secured, and viscosity and surface tension are prevented from being excessively increased, so that suitable electrolyte impregnation properties may be obtained.

## Lithium secondary battery

Next, a lithium secondary battery according to the present invention will be described.

The lithium secondary battery according to the present invention includes a positive electrode including a positive electrode active material, a negative electrode including a negative electrode active material, a separator interposed between the positive electrode and the negative electrode, and a non-aqueous electrolyte solution, wherein the non-aqueous electrolyte solution is the non-aqueous electrolyte solution according to the present invention. The non-aqueous electrolyte solution has been described above, and thus, the description thereof will be omitted, and hereinafter, the other components will be described.

## Positive electrode

The positive electrode according to the present invention includes a positive electrode active material, and may be manufactured by coating a positive electrode slurry including the positive electrode active material, a binder, a conductive material, a solvent, and the like on a positive electrode current collector, followed by drying and roll-pressing.

The positive electrode current collector is not particularly limited as long as it has conductivity without causing a chemical change in the battery. For example, stainless steel; aluminum; nickel; titanium; fired carbon; or aluminum or stainless steel that is surface-treated with one of carbon, nickel, titanium, silver, and the like may be used.

The positive electrode active material is a compound capable of reversible intercalation and de-intercalation of lithium, and may be one or more selected from the group consisting of LCO($LiCoO_2$), LNO($LiNiO_2$), LMO($LiMnO_2$), $LiMn_2O_4$, $LiCoPO_4$, LFP($LiFePO_4$), or $LiNi_{1-x-y-z}Co_xM^1_yM^1_yM^2_zO_2$ ($M^1$ and $M^2$ are each independently any one selected from the group consisting of Al, Ni, Co, Fe, Mn, V, Cr, Ti, W, Ta, Mg, and Mo, and x, y and z are each independently an atomic fraction of an oxide composition element, wherein $0 \leq x < 0.5$, $0 \leq y < 0.5$, $0 \leq z < 0.5$, and $x+y+z=1$) including $LiNiMnCoO_2$ and the like.

In an embodiment of the present invention, the positive electrode active material may be represented by Formula 2 or Formula 3 below.

[Formula 2] $Li\ (Ni_aCo_bMn_cM_d)O_2$

In Formula 2, M is W, Cu, Fe, V, Cr, Ti, Zr, Zn, Al, In, Ta, Y, La, Sr, Ga, Sc, Gd, Sm, Ca, Ce, Nb, Mg, B, or Mo, and a, b, c, and d are each an atomic fraction of an independent element, wherein $0.50 \le a \le 0.95$, $0.025 \le b \le 0.25$, $0.025 \le c \le 0.25$, $0 \le d \le 0.05$, and a+b+c+d=1.

[Formula 3] $LiFe_{1-e}M'_ePO_4$

In Formula 3, M' is one or more selected from Ni, Co, Mn, Al, Mg, Y, Zn, In, Ru, Sn, Sb, Ti, Te, Nb, Mo, Cr, Zr, W, Ir, and V, and $0 \le e < 1$.

Preferably, the a, the b, the c, and the d of Formula 2 may respectively be $0.60 \le a \le 0.90$, $0.05 \le b \le 0.20$, $0.05 \le c \le 0.20$, and $0 \le d \le 0.03$.

When the positive electrode active material of the present invention is an NCM positive electrode active material including nickel (Ni), cobalt (Co), and manganese (Mn), higher energy density may be implemented by increasing the content of Ni, but there is a disadvantage in that positive electrode surface reactivity and stability are deteriorated. However, the disadvantage may be overcome when aluminum (Al) is introduced as M.

Preferably, the e of Formula 3 may be 0.

The positive electrode active material may be included in an amount of 80 wt% to 99 wt%, specifically 90 wt% to 99 wt% based on the total weight of solids in a positive electrode slurry. At this time, when the content of the positive electrode active material is 80 wt% or less, energy density is lowered to lower capacity.

The binder is a component for assisting in bonding of an active material and a conductive material, and in bonding to a current collector, and may be typically added in an amount of 1 wt% to 30 wt% based on the total weight of solids in a positive electrode slurry. Examples of the binder may include polyvinylidene fluoride, polyvinyl alcohol, carboxymethyl cellulose (CMC), starch, hydroxypropyl cellulose, regenerated cellulose, polyvinylpyrrolidone, polytetrafluoroethylene, polyethylene, polypropylene, an ethylene-propylene-diene monomer, a sulfonated ethylene-propylene-diene monomer, styrene-butadiene rubber, fluorine rubber, or various copolymers thereof.

In addition, the conductive material is a material imparting conductivity without causing a chemical change in the battery, and may be added in an amount of 0.5 wt% to 20 wt% based on the total weight of solids in a positive electrode slurry.

Examples of the conductive material may include carbon black such as acetylene black, Ketjen black, channel black, furnace black, lamp black, or thermal black; graphite powder of natural graphite, artificial graphite, carbon nanotubes, or graphite, which has a very developed crystal structure; conductive fiber such as carbon fiber or metal fiber; conductive powder such as fluorocarbon powder, aluminum powder, or nickel powder; a conductive whisker such as zinc oxide and potassium titanate; a conductive metal oxide such as titanium oxide; or a conductive material such as a polyphenylene derivative, and the like.

In addition, a solvent of the positive electrode slurry may include an organic solvent such as N-methyl-2-pyrrolidone (NMP), and may be used in an amount such that a preferred viscosity is achieved when the positive electrode active material, the binder, the conductive material, and the like are included. For example, the solvent may be included in an amount such that the concentration of solids in a positive electrode slurry including a positive electrode active material, a binder, and a conductive material is 40 wt% to 99 wt%, preferably 50 wt% to 99 wt%.

**(2) Negative electrode**

The negative electrode according to the present invention includes a negative electrode active material, and may be manufactured by coating a negative electrode slurry including the negative electrode active material, a binder, a conductive material, a solvent, and the like on a negative electrode current collector, followed by drying and roll-pressing.

The negative electrode current collector typically has a thickness of 3 μm to 500 μm. The negative electrode current collector is not particularly limited as long as it has high conductivity without causing a chemical change in the battery. For example, copper; stainless steel; aluminum; nickel; titanium; fired carbon, copper or stainless steel that is surface-treated with one of carbon, nickel, titanium, silver, and the like, or an aluminum-cadmium alloy and the like may be used. Also, as in the case of the positive electrode current collector, microscopic irregularities may be formed on the surface of the negative electrode current collector to improve the coupling force of a negative electrode active material, and the negative electrode current collector may be used in various forms of such as a film, a sheet, a foil, a net, a porous body, a foam body, and a non-woven fabric body.

In addition, the negative electrode active material may include one or more selected from a carbon material

capable of reversible intercalation/de-intercalation of lithium ions; a metal or an alloy of the metal and lithium; a metal complex oxide; a material capable of doping and undoping lithium; a lithium metal; and a transition metal oxide.

As the carbon material capable of reversible intercalation/de-intercalation of lithium metals, a carbon-based negative electrode active material commonly used in a lithium ions may be used without particular limitation, and examples thereof may include a crystalline carbon, an amorphous carbon, or a combination thereof. Examples of the crystalline carbon may include graphite such as an irregular, planar, flaky, spherical, or fibrous natural graphite or artificial graphite, and examples of the amorphous carbon may include soft carbon (low-temperature fired carbon), hard carbon, mezophase pitch carbides, fired cokes, and the like.

As the metal or the alloy of the metal and lithium, a metal selected from the group consisting of Cu, Ni, Na, K, Rb, Cs, Fr, Be, Mg, Ca, Sr, Si, Sb, Pb, In, Zn, Ba, Ra, Ge, Al, and Sn, or an alloy of the metal and lithium may be used.

As the metal composite oxide, one selected from the group consisting of PbO, $PbO_2$, $Pb_2O_3$, $Pb_3O_4$, $Sb_2O_3$, $Sb_2O_4$, $Sb_2O_5$, GeO, $GeO_2$, $Bi_2O_3$, $Bi_2O_4$, $Bi_2O_5$, $Li_X Fe_2O_3(0 \le x \le 1)$, $Li_X WO_2(0 \le x \le 1)$, and $Sn_xMe_{1-X} Me'_Y O_Z$ (Me: Mn, Fe, Pb, Ge; Me'; an element each in Group 1, Group 2, and Group 3 of the periodic table, halogen: $0<x\le1$; $1\le y\le3$; $1\le z\le8$) may be used.

The material capable of doping and undoping lithium may be Si, $SiO_x(0<x\le2)$, an Si-Y alloy (wherein Y is an element selected from the group consisting of an alkali metal, an alkaline earth metal, a Group 13 element, a Group 14 element, a transition metal, a rare earth element, and a combination thereof, but not Si), Sn, $SnO_2$, Sn-Y (wherein Y is an element selected from the group consisting of an alkali metal, an alkaline earth metal, a Group 13 element, a Group 14 element, a transition metal, a rare earth element, and a combination thereof, but not Sn), and the like, or at least one thereof may be mixed with $SiO_2$ and used. The element Y may be selected from the group consisting of Mg, Ca, Sr, Ba, Ra, Sc, Y, Ti, Zr, Hf, Rf, V, Nb, Ta, Db(dubnium), Cr, Mo, W, Sg, Tc, Re, Bh, Fe, Pb, Ru, Os, Hs, Rh, Ir, Pd, Pt, Cu, Ag, Au, Zn, Cd, B, Al, Ga, Sn, In, Ge, P, As, Sb, Bi, S, Se, Te, Po, and a combination thereof.

Examples of the transition metal oxide include a lithium-containing titanium composite oxide (LTO), a vanadium oxide, a lithium vanadium oxide, and the like.

The negative electrode active material may be included in an amount of 80 wt% to 99 wt% based on the total weight of solids in a negative electrode slurry.

The binder is a component for assisting in bonding among a conductive material, an active material, and a current collector, and may be typically added in an amount of 1 wt% to 30 wt% based on the total weight of solids in a negative electrode slurry. Examples of the binder may include polyvinylidene fluoride, polyvinyl alcohol, carboxymethyl cellulose (CMC), starch, hydroxypropyl cellulose, regenerated cellulose, polyvinylpyrrolidone, polytetrafluoroethylene, polyethylene, polypropylene, an ethylene-propylene-diene monomer, a sulfonated ethylene-propylene-diene monomer, styrene-butadiene rubber, fluorine rubber, or various copolymers thereof.

The conductive material is a component for further improving the conductivity of a negative electrode active material, and may be added in an amount of 0.5 wt% to 20 wt% based on the total weight of solids in a negative electrode slurry. The conductive material is not particularly limited as long as it has conductivity without causing a chemical change in the battery, and for example, carbon black such as acetylene black, Ketjen black, channel black, furnace black, lamp black, or thermal black; graphite powder of natural graphite, artificial graphite, carbon nanotubes, or graphite, which has a very developed crystal structure; conductive fiber such as carbon fiber or metal fiber; conductive powder such as fluorocarbon powder, aluminum powder, or nickel powder; a conductive whisker such as zinc oxide and potassium titanate; a conductive metal oxide such as titanium oxide; or a conductive material such as a polyphenylene derivative, and the like.

A solvent of the negative electrode slurry may include water, or an organic solvent such as NMP, an alcohol, or the like, and may be used in an amount such that a preferred viscosity is achieved when the negative electrode active material, the binder, the conductive material, and the like are included. For example, the solvent may be included in an amount such that the concentration of solids in a slurry including a negative electrode active material, a binder and a conductive material is 30 wt% to 99 wt%, preferably 40 wt% to 99 wt%.

**(3) Separator**

The lithium secondary battery according to the present invention includes a separator between the positive electrode and the negative electrode.

The separator is to separate the negative electrode and the positive electrode and to provide a movement path for lithium ions. Any separator typically used as a separator in a lithium secondary battery may be used without particular limitation, and particularly, a separator which has low resistance to ion movement of an electrolyte solution, has excellent electrolyte solution impregnation, and which is safe is preferable.

Specifically, as a separator, a porous polymer film, for example, a porous polymer film manufactured using a polyolefin-based polymer such as an ethylene homopolymer, a propylene homopolymer, an ethylene/butene copolymer, an ethylene/hexene copolymer, and an ethylene/methacrylate copolymer, or a laminated structure having two or more

layers thereof may be used. Also, a typical porous non-woven fabric, for example, a non-woven fabric formed of glass fiber having a high melting point, polyethylene terephthalate fiber, or the like may be used. Also, a coated separator including a ceramic component or a polymer material may be used to secure heat resistance or mechanical strength, and may be used in a single-layered or a multi-layered structure.

The lithium secondary battery according to the present invention as described above may be usefully used in portable devices such as a mobile phone, a notebook computer, and a digital camera, and in electric cars such as a hybrid electric vehicle (HEV).

Accordingly, according to another embodiment of the present invention, a battery module including the lithium secondary battery as a unit cell, and a battery pack including the battery module are provided.

The battery module or the battery pack may be used as a power source of one or more medium-and-large-sized devices, for example, a power tool, an electric car including an electric vehicle (EV), a hybrid electric vehicle, and a plug-in hybrid electric vehicle (PHEV), and a power storage system.

The external shape of the lithium secondary battery of the present invention is not particularly limited, but may be a cylindrical shape using a can, a square shape, a pouch shape, a coin shape, or the like.

The lithium secondary battery according to the present invention may be used in a battery cell which is used as a power source for a small-sized device, and may also be preferably used as a unit cell in a medium-and-large-sized battery module including a plurality of battery cells.

Hereinafter, the present invention will be described in detail with reference to specific examples.

MODE FOR CARRYING OUT THE INVENTION

**<Examples: Manufacturing of lithium secondary battery>**

**Example 1.**

(Preparation of non-aqueous electrolyte solution)

Ethylene carbonate (EC) : ethylmethyl carbonate (EMC) were mixed in a volume ratio of 30:70, and then $LiPF_6$ was dissolved therein to 1.0 M to prepare a non-aqueous organic solution. 0.5 g of a compound represented by Formula 1-1, 0.5 g of vinylene carbonate, and the remainder of the non-aqueous organic solution were mixed to prepare 100 g of a non-aqueous electrolyte solution.

(Manufacturing of lithium secondary battery)

To N-methyl-2-pyrrolidone (NMP), $Li[Ni_{0.86}Co_{0.05}Mn_{0.07}Al_{0.02}]O_2$(NCMA) as a positive electrode active material, a conductive material (carbon black), and a binder (polyvinylidene fluoride) were added at a weight ratio of 97.5:1:1.5 to prepare a positive electrode slurry (solid content: 60 wt%). The positive electrode slurry was applied and dried on an aluminum (Al) thin film having a thickness of about 15 μm as a positive electrode current collector, and then roll pressing was performed thereon to manufacture a positive electrode.

In addition, as a negative electrode active material, graphite in which artificial graphite and natural graphite were blended at a weight ratio of 8:2, styrene-butadiene rubber (SBR) as a binder, sodium carboxymethyl cellulose (CMC) as a thickener, and carbon black as a conductive material were mixed at a weight ratio of 96.3:1:1.5:1.2, and then added to the NMP solvent to prepare a negative electrode mixture slurry. The negative electrode mixture slurry was applied on a copper (Cu) thin film having a thickness of about 10 μm as a negative electrode current collector, dried and then roll pressed to manufacture a negative electrode.

In a dry room, a separator was interposed between the positive electrode and the negative electrode, and then the prepared non-aqueous electrolyte solution was injected thereto to manufacture a coin half-cell type lithium secondary battery.

**Example 2.**

A lithium secondary battery was manufactured in the same manner as in Example 1 except that the content of the compound represented by Formula 1-1 was changed to 1 g when preparing a non-aqueous electrolyte solution.

**Example 3.**

A lithium secondary battery was manufactured in the same manner as in Example 1 except that $LiFePO_4$(LFP) was used as a positive electrode active material instead of NCMA when manufacturing a lithium secondary battery.

**Example 4.**

A lithium secondary battery was manufactured in the same manner as in Example 2 except that $LiFePO_4$(LFP) was used as a positive electrode active material instead of NCMA when manufacturing a lithium secondary battery.

**Example 5.**

A lithium secondary battery was manufactured in the same manner as in Example 1 except that the content of the compound represented by Formula 1-1 was changed to 5 g when preparing a non-aqueous electrolyte solution.

**Example 6.**

A lithium secondary battery was manufactured in the same manner as in Example 1 except that the content of the compound represented by Formula 1-1 was changed to 0.1 g when preparing a non-aqueous electrolyte solution.

**Comparative Example 1.**

A lithium secondary battery was manufactured in the same manner as in Example 1 except that the compound represented by Formula 1-1 was not added when preparing a non-aqueous electrolyte solution.

**Comparative Example 2.**

A lithium secondary battery was manufactured in the same manner as in Example 1 except that a compound represented by Formula B-1 below was added instead of the compound represented by Formula 1-1 when preparing a non-aqueous electrolyte solution.

[Formula B-1]

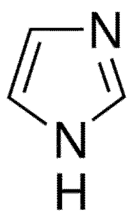

**Comparative Example 3.**

A lithium secondary battery was manufactured in the same manner as in Comparative Example 2 except that the content of the compound represented by Formula B-1 was changed to 1 g when preparing a non-aqueous electrolyte solution.

**Comparative Example 4.**

A lithium secondary battery was manufactured in the same manner as in Example 1 except that a compound represented by Formula B-2 below was added instead of the compound represented by Formula 1-1 when preparing a non-aqueous electrolyte solution.

[Formula B-2]

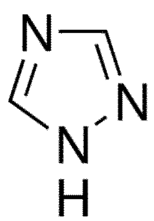

**Comparative Example 5.**

A lithium secondary battery was manufactured in the same manner as in Comparative Example 4 except that the content of the compound represented by Formula B-2 was changed to 1 g when preparing a non-aqueous electrolyte solution.

**Comparative Example 6.**

A lithium secondary battery was manufactured in the same manner as in Comparative Example 2 except that $LiFePO_4$(LFP) was used as a positive electrode active material instead of NCMA when manufacturing a lithium secondary battery.

**Comparative Example 7.**

A lithium secondary battery was manufactured in the same manner as in Comparative Example 3 except that $LiFePO_4$(LFP) was used as a positive electrode active material instead of NCMA when manufacturing a lithium secondary battery.

**Comparative Example 8.**

A lithium secondary battery was manufactured in the same manner as in Example 1 except that a compound represented by Formula B-3 below was added instead of the compound represented by Formula 1-1 when preparing a non-aqueous electrolyte solution.

[Formula B-3]

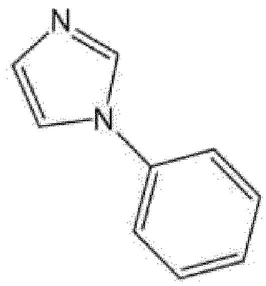

**Comparative Example 9.**

A lithium secondary battery was manufactured in the same manner as in Comparative Example 8 except that the content of the compound represented by Formula B-3 was changed to 1 g when preparing a non-aqueous electrolyte solution.

**Comparative Example 10.**

A lithium secondary battery was manufactured in the same manner as in Example 1 except that a compound represented by Formula B-4 below was added instead of the compound represented by Formula 1-1 when preparing a non-aqueous electrolyte solution.

[Formula B-4]

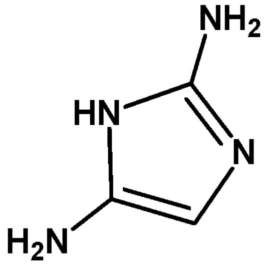

**<Experimental Examples: Evaluation of performance of lithium secondary battery>**

**Experimental Example 1. Evaluation of high-temperature (45°C) lifespan properties**

(1) Measurement of initial resistance and resistance increase rate (%)

The lithium secondary batteries manufactured in Examples and Comparative Examples were each activated with 0.1 C CC, and then degassed.

Thereafter, under the condition of constant current-constant voltage (CC-CV) charging at 25°C, the lithium secondary batteries were each charged to 4.20 V with 0.33C CC, followed by a 0.05 C current cut, and then discharged to 2.5 V with 0.33 C under the condition of CC. The above charging/discharging was set to one cycle, and three cycles were performed, and then DC-iR was calculated through a voltage drop that appeared when a discharge pulse was applied for 10 seconds at 2.5 C after charging to 50% of state of charge (SOC), and the measured resistance was defined as an initial resistance. The voltage drop was measured using the PNE-0506 charger and discharger (Manufacturer: PNE solution, 5V, 6A).

Thereafter, after performing 200 cycles of charging/discharging at a high temperature (45°C) under the same charging/discharging conditions as the above, DC-iR was calculated through a voltage drop that appeared when a discharge pulse was applied for 10 seconds at 2.5 C after charging to 50% of state of charge (SOC) . The resistance increase rate (%) after 200 cycles calculated by substituting the above into Equation (1) below is shown in Table 1 below.

Resistance increase rate (%) = {(resistance after 200 cycles-initial resistance)/initial resistance} $\times$ 100

(2) Measurement of capacity retention rate (%)

The lithium secondary batteries manufactured in Examples and Comparative Examples were each activated with 0.1 C CC, and then degassed. Thereafter, under the condition of constant current-constant voltage (CC-CV) charging at 25°C, the lithium secondary batteries were each charged to 4.20 V with 0.33C CC, followed by a 0.05 C current cut, and then discharged to 2.5 V with 0.33 C under the condition of CC.

Next, under the condition of constant current-constant voltage (CC-CV) charging at 45°C, the lithium secondary batteries were each charged to 4.20 V with 0.33C CC, followed by a 0.05 C current cut, and then discharged to 2.5 V with 0.33 C under the condition of CC. The above charging/discharging was set to one cycle, and 200 cycles of charging/discharging were performed at a high temperature (45°C), during which a discharge capacity was measured using the PNE-0506 charger and discharger (Manufacturer: PNE solution, 5V, 6A). The capacity retention rate was calculated by substituting the measured discharge capacity into Equation (2) below, and results are shown in Table 1 below.

Capacity retention rate (%) = (discharge capacity after 200 cycles/discharge capacity after 1 cycle) $\times$ 100

**Experimental Example 2. High-temperature storage properties evaluation**

Each of the lithium secondary batteries manufactured in Examples and Comparative Examples was fully charged to 100% of SOC with 4.2 V (0.05 C cut off) under the conditions of CC/CV and 0.33 C at 25°C. Thereafter, the fully-charged lithium secondary battery was stored at a high temperature (60°C) for 12 weeks to measure the capacity retention

rate, resistance increase rate, and volume increase rate thereof, and the results are shown in Table 1 below.

At this time, the capacity retention rate was calculated by substituting the discharge capacity of the lithium secondary battery measured before the high-temperature storage and the discharge capacity of the lithium secondary battery measured after the high-temperature storage, which were measured by using the PNE-0506 charger and discharger (Manufacturer: PNE solution, 5V, 6A), into Equation (3) below, the resistance increase rate was calculated by substituting the initial resistance value measured before the high-temperature storage and the resistance value measured after the high-temperature storage into Equation (4) below, and the volume increase rate was calculated by substituting the initial volume before the high-temperature storage and the volume after the high-temperature storage, which were measured in a buoyancy manner, into Equation (5) below.

Capacity retention rate (%) = (discharge capacity after high-temperature storage/discharge capacity before high-temperature storage) × 100

Resistance increase rate (%) = {(resistance value after high-temperature storage-initial resistance value)/initial resistance value} × 100

Volume increase rate (%) = {(volume after high-temperature storage-initial volume)/initial volume} × 100

[Table 1]

| | Positive electrode active material | First additive | | Experimental Example 1 | | | Experimental Example 2 | | |
|---|---|---|---|---|---|---|---|---|---|
| | | Chemical Formula | Content (g) | Initial resistance (Ohm) | Resistance increase rate (%) | Capacity retention rate (%) | Capacity retention rate (%) | Resistance increase rate (%) | Volume increase rate (%) |
| Example 1 | NCMA | 1-1 | 0.5 | 5.45 | 1.2 | 98.5 | 97.5 | 3.5 | 5.1 |
| Example 2 | NCMA | 1-1 | 1 | 5.67 | 1.12 | 98 | 97.3 | 3.8 | 4 . 8 |
| Example 3 | LFP | 1-1 | 0.5 | 5.12 | 1.3 | 98.2 | 96.5 | 3.7 | 3.2 |
| Example 4 | LFP | 1-1 | 1 | 5.25 | 1.15 | 97.8 | 96.2 | 4.1 | 3 |
| Example 5 | NCMA | 1-1 | 5 | 6.12 | 1.02 | 97.2 | 94.2 | 3.5 | 4.1 |
| Example 6 | NCMA | 1-1 | 0.1 | 5.31 | 2.5 | 98.7 | 96.5 | 5.4 | 7 . 6 |
| Comparative Example 1 | NCMA | - | - | 8.75 | 32.4 | 79.8 | 75.2 | 34.5 | 24.7 |
| Comparative Example 2 | NCMA | B-1 | 0.5 | 5.67 | 13.4 | 91.2 | 88.5 | 12.4 | 15.4 |
| Comparative Example 3 | NCMA | B-1 | 1 | 5.98 | 13.2 | 90.8 | 87.9 | 12.8 | 14.7 |
| Comparative Example 4 | NCMA | B-2 | 0.5 | 5.57 | 15.5 | 92.1 | 85.4 | 13.5 | 17.5 |
| Comparative Example 5 | NCMA | B-2 | 1 | 5.72 | 14.7 | 91.5 | 84.7 | 14.1 | 18.4 |
| Comparative Example 6 | LFP | B-1 | 0.5 | 5.32 | 14.1 | 90.7 | 87.5 | 12.9 | 14.2 |
| Comparative Example 7 | LFP | B-1 | 1 | 5.49 | 13.5 | 90.1 | 86.8 | 13.5 | 13.4 |

(continued)

| | Positive electrode active material | First additive | | Experimental Example 1 | | | Experimental Example 2 | | |
|---|---|---|---|---|---|---|---|---|---|
| | | Chemical Formula | Content (g) | Initial resistance (Ohm) | Resistance increase rate (%) | Capacity retention rate (%) | Capacity retention rate (%) | Resistance increase rate (%) | Volume increase rate (%) |
| Comparative Example 8 | NCMA | B-3 | 0.5 | 5.62 | 17.5 | 91.1 | 83.2 | 14.2 | 20.1 |
| Comparative Example 9 | NCMA | B-3 | 1 | 5.98 | 15.2 | 89.5 | 82.7 | 15.3 | 19.6 |
| Comparative Example 10 | NCMA | B-4 | 0.5 | 5. 97 | 20.1 | 88.7 | 82.4 | 15.5 | 19.9 |

Through the results in Table 1, it can be confirmed that when the compound represented by Formula (1) and vinylene carbonate are included as an electrolyte additive at the same time, both high-temperature lifespan and storage properties are excellent.

Specifically, it can be confirmed that Examples 1 to 6 have excellent high-temperature lifespan and storage properties not only compared to Comparative Example 1 not using the compound represented by Formula 1 as an additive, but also compared to cases in which the compound represented by Formula B-1 having an imidazole structure in which an amino group was not substituted was used instead of the compound represented by Formula 1 (Comparative Examples 2, 3, 6, and 7), cases in which the compound represented by Formula B-2 having a triazole structure was used (Comparative Examples 4 and 5), cases in which the compound represented by Formula B-3 having a phenylimidazole structure was used (Comparative Examples 8 and 9), a case in which the compound represented by Formula B-4 having a structure in which two amino groups were substituted was used (Comparative Example 10) .

In addition, it can be seen that Examples 1 to 4, and 6 in which the content of the compound represented by Formula 1 is 3 wt% or less are more advantageous in terms of initial resistance and capacity retention rate after the high-temperature storage than Example 5 in which the content of the same is greater than 3 wt%, and Examples 1 to 5 in which the content of the compound represented by Formula 1 is 0.2 wt% or greater are more advantageous in terms of resistance increase rate and volume increase rate after the high-temperature storage than Example 6 in which the content of the same is less than 0.2 wt%.

## Claims

1. A non-aqueous electrolyte solution for a lithium secondary battery comprising:

   a lithium salt;
   an organic solvent; and
   a first additive which is a compound represented by Formula 1 below:

   [Formula 1]

   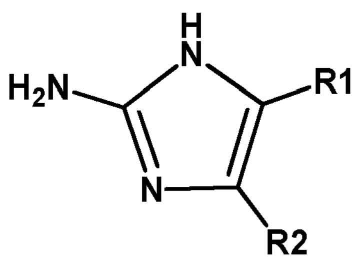

   wherein in Formula 1,
   R1, and R2 are each independently hydrogen; or an alkyl group having 1 to 5 carbon atoms.

2. The non-aqueous electrolyte solution of claim 1, wherein R1 and R2 are each hydrogen.

3. The non-aqueous electrolyte solution of claim 1, further comprising one or more second additives selected from vinylene carbonate and vinylethylene carbonate.

4. The non-aqueous electrolyte solution of claim 1, wherein the content of the first additive is 0.1 wt% to 5 wt% based on the total weight of the non-aqueous electrolyte solution.

5. The non-aqueous electrolyte solution of claim 1, wherein the content of the first additive is 0.1 wt% to 3 wt% based on the total weight of the non-aqueous electrolyte solution.

6. The non-aqueous electrolyte solution of claim 3, wherein the content of the second additive is 0.1 wt% to 5 wt% based on the total weight of the non-aqueous electrolyte solution.

7. The non-aqueous electrolyte solution of claim 3, wherein the weight ratio of the first additive and the second additive is 1:0.2 to 1:1.5.

8. The non-aqueous electrolyte solution of claim 3, wherein the weight ratio of the first additive and the second additive is 1:0.5 to 1:1.

9. The non-aqueous electrolyte solution of claim 1, wherein the concentration of the lithium salt is 0.3 M to 3.0 M.

10. The non-aqueous electrolyte solution of claim 1, wherein the organic solvent comprises a cyclic carbonate-based solvent and a linear carbonate-based solvent.

11. The non-aqueous electrolyte solution of claim 10, wherein the volume ratio of the cyclic carbonate-based solvent and the linear carbonate-based solvent is 3:7 to 2:8.

12. A lithium secondary battery comprising:

a positive electrode including a positive electrode active material;
a negative electrode including a negative electrode active material;
a separator interposed between the positive electrode and the negative electrode; and
the non-aqueous electrolyte solution of claim 1.

13. The lithium secondary battery of claim 12, wherein the positive electrode active material comprises a lithium composite transition metal oxide represented by Formula 2 or Formula 3 below:

[Formula 2] $Li(Ni_aCo_bMn_cM_d)O_2$

wherein in Formula 2,

M is W, Cu, Fe, V, Cr, Ti, Zr, Zn, Al, In, Ta, Y, La, Sr, Ga, Sc, Gd, Sm, Ca, Ce, Nb, Mg, B, or Mo, and
a, b, c, and d are each an atomic fraction of an independent element, wherein $0.50 \leq a \leq 0.95$, $0.025 \leq b \leq 0.25$, $0.025 \leq c \leq 0.25$, $0 \leq d \leq 0.05$, and $a+b+c+d=1$, and

[Formula 3] $LiFe_{1-e}M'_ePO_4$

wherein in Formula 3,
M' is one or more selected from Ni, Co, Mn, Al, Mg, Y, Zn, In, Ru, Sn, Sb, Ti, Te, Nb, Mo, Cr, Zr, W, Ir, and V, and

$$0 \leq e < 1.$$

**Patentansprüche**

1. Nichtwässrige Elektrolytlösung für eine Lithiumsekundärbatterie, umfassend:

ein Lithiumsalz;
ein organisches Lösungsmittel; und
ein erstes Additiv, das eine durch nachstehende Formel 1 dargestellte Verbindung ist:

[Formel 1]

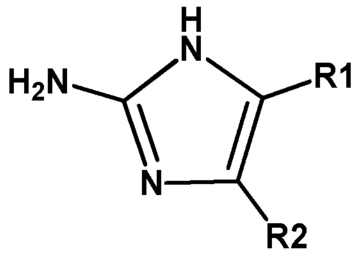

worin, in Formel 1,
R1 und R2 jeweils unabhängig Wasserstoff; oder eine Alkylgruppe mit 1 bis 5 Kohlenstoffatomen sind.

**2.** Nichtwässrige Elektrolytlösung gemäß Anspruch 1, worin R1 und R2 jeweils Wasserstoff sind.

**3.** Nichtwässrige Elektrolytlösung gemäß Anspruch 1, ferner umfassend ein oder mehrere zweite Additive, ausgewählt aus Vinylencarbonat und Vinylethylencarbonat.

**4.** Nichtwässrige Elektrolytlösung gemäß Anspruch 1, worin der Gehalt des ersten Additivs, basierend auf dem Gesamtgewicht der nichtwässrigen Elektrolytlösung, 0,1 Gew.-% bis 5 Gew.-% beträgt.

**5.** Nichtwässrige Elektrolytlösung gemäß Anspruch 1, worin der Gehalt des ersten Additivs, basierend auf dem Gesamtgewicht der nichtwässrigen Elektrolytlösung, 0,1 Gew.-% bis 3 Gew.-% beträgt.

**6.** Nichtwässrige Elektrolytlösung gemäß Anspruch 3, worin der Gehalt des zweiten Additivs, basierend auf dem Gesamtgewicht der nichtwässrigen Elektrolytlösung, 0,1 Gew.-% bis 5 Gew.-% beträgt.

**7.** Nichtwässrige Elektrolytlösung gemäß Anspruch 3, worin das Gewichtsverhältnis des ersten Additivs und des zweiten Additivs 1:0,2 bis 1:1,5 beträgt.

**8.** Nichtwässrige Elektrolytlösung gemäß Anspruch 3, worin das Gewichtsverhältnis des ersten Additivs und des zweiten Additivs 1:0,5 bis 1:1 beträgt.

**9.** Nichtwässrige Elektrolytlösung gemäß Anspruch 1, worin die Konzentration des Lithiumsalzes 0,3 M bis 3,0 M beträgt.

**10.** Nichtwässrige Elektrolytlösung gemäß Anspruch 1, worin das organische Lösungsmittel ein Lösungsmittel auf Basis eines cyclischen Carbonats und ein Lösungsmittel auf Basis eines linearen Carbonats umfasst.

**11.** Nichtwässrige Elektrolytlösung gemäß Anspruch 10, worin das Volumenverhältnis des Lösungsmittels auf Basis eines cyclischen Carbonats und des Lösungsmittels auf Basis eines linearen Carbonats 3:7 bis 2:8 beträgt.

**12.** Lithiumsekundärbatterie, umfassend:

eine positive Elektrode, die ein Aktivmaterial für eine positive Elektrode enthält;
eine negative Elektrode, die ein Aktivmaterial für eine negative Elektrode enthält;
einen Separator, der zwischen der positiven Elektrode und der negativen Elektrode bereitgestellt ist; und
die nichtwässrige Elektrolytlösung gemäß Anspruch 1.

**13.** Lithiumsekundärbatterie gemäß Anspruch 12, worin das Aktivmaterial für eine positive Elektrode ein durch nachstehende Formel 2 oder Formel 3 dargestelltes Lithium-Komposit-Übergangsmetalloxid umfasst:

[Formel 2] $Li(Ni_aCo_bMn_cM_d)O_2$

worin, in Formel 2,

M W, Cu, Fe, V, Cr, Ti, Zr, Zn, Al, In, Ta, Y, La, Sr, Ga, Sc, Gd, Sm, Ca, Ce, Nb, Mg, B oder Mo ist, und a, b, c und d jeweils der atomare Anteil eines unabhängigen Elements ist, worin $0{,}50 \leq a \leq 0{,}95$, $0{,}025 \leq b \leq 0{,}25$, $0{,}025 \leq c \leq 0{,}25$, $0 \leq d \leq 0{,}05$ und $a+b+c+d=1$, und

[Formel 3] $LiFe_{1-e}M'_{e}PO_4$

worin, in Formel 3,
M' eines oder mehrere ist, ausgewählt aus Ni, Co, Mn, Al, Mg, Y, Zn, In, Ru, Sn, Sb, Ti, Te, Nb, Mo, Cr, Zr, W, Ir und V, und

$$0 \leq e < 1.$$

**Revendications**

1. Solution électrolytique non aqueuse pour une batterie secondaire au lithium comprenant :

un sel de lithium ;
un solvant organique ; et
un premier additif qui est un composé représenté par la Formule 1 ci-dessous :

[Formule 1]

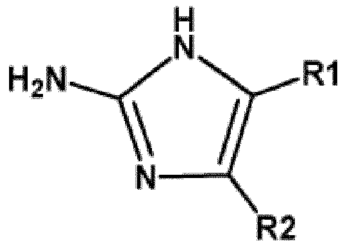

dans laquelle dans la Formule 1,
R1 et R2 sont chacun indépendamment un hydrogène ; ou un groupe alkyle ayant de 1 à 5 atomes de carbone.

2. Solution électrolytique non aqueuse selon la revendication 1, dans laquelle R1 et R2 sont chacun un hydrogène.

3. Solution électrolytique non aqueuse selon la revendication 1, comprenant en outre un ou plusieurs seconds additifs sélectionnés parmi le carbonate de vinylène et le carbonate de vinyléthylène.

4. Solution électrolytique non aqueuse selon la revendication 1, dans laquelle la teneur du premier additif est de 0,1 % en poids à 5 % en poids sur la base du poids total de la solution électrolytique non aqueuse.

5. Solution électrolytique non aqueuse selon la revendication 1, dans laquelle la teneur du premier additif est de 0,1 % en poids à 3 % en poids sur la base du poids total de la solution électrolytique non aqueuse.

6. Solution électrolytique non aqueuse selon la revendication 3, dans laquelle la teneur du second additif est de 0,1 % en poids à 5 % en poids sur la base du poids total de la solution électrolytique non aqueuse.

7. Solution électrolytique non aqueuse selon la revendication 3, dans laquelle le rapport en poids entre le premier additif et le second additif est de 1:0,2 à 1:1,5.

8. Solution électrolytique non aqueuse selon la revendication 3, dans laquelle le rapport en poids entre le premier additif et le second additif est de 1:0,5 à 1:1.

**9.** Solution électrolytique non aqueuse selon la revendication 1, dans laquelle la concentration du sel de lithium est de 0,3 M à 3,0 M.

**10.** Solution électrolytique non aqueuse selon la revendication 1, dans laquelle le solvant organique comprend un solvant à base de carbonate cyclique et un solvant à base de carbonate linéaire.

**11.** Solution électrolytique non aqueuse selon la revendication 10, dans laquelle le rapport de volume entre le solvant à base de carbonate cyclique et le solvant à base de carbonate linéaire est de 3:7 à 2:8.

**12.** Batterie secondaire au lithium comprenant :

une électrode positive incluant un matériau actif d'électrode positive ;
une électrode négative incluant un matériau actif d'électrode négative ;
un séparateur interposé entre l'électrode positive et l'électrode négative ; et
la solution électrolytique non aqueuse selon la revendication 1.

**13.** Batterie secondaire au lithium selon la revendication 12, dans laquelle le matériau actif d'électrode positive comprend un oxyde de métal de transition composite de lithium représenté par la Formule 2 ou la Formule 3 ci-dessous :

[Formule 2] $Li(Ni_aCo_bMn_cM_d)O_2$

dans laquelle dans la Formule 2,

M est W, Cu, Fe, V, Cr, Ti, Zr, Zn, Al, In, Ta, Y, La, Sr, Ga, Sc, Gd, Sm, Ca, Ce, Nb, Mg, B, ou Mo, et
a, b, c et d sont chacun une fraction atomique d'un élément indépendant, dans laquelle $0{,}50 \leq a \leq 0{,}95$, $0{,}025 \leq b \leq 0{,}25$, $0{,}025 \leq c \leq 0{,}25$, $0 \leq d \leq 0{,}05$, et $a+b+c+d=1$, et

[Formule 3] $LiFe_{1-e}M'_ePO_4$

dans laquelle dans la Formule 3,
M' est un ou plusieurs éléments sélectionnés parmi Ni, Co, Mn, Al, Mg, Y, Zn, In, Ru, Sn, Sb, Ti, Te, Nb, Mo, Cr, Zr, W, Ir et V, et

$$0 \leq e < 1.$$

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020210037733
- JP 2003123834 A
- KR 101459402 B1
- KR 20170048915 A
- JP 2004207451 A
- JP 2007095983 A